# EUROPEAN PATENT APPLICATION

(11) **EP 1 529 850 A1**
(43) Date of publication of application: **11.05.2005**
(21) Application number: 03025672.1
(22) Date of filing: 07.11.2003
(51) Int. Cl.: C14C 15/00, C02F 3/28, C12P 5/02, B09B 3/00

(54) **Process for treating tanned leather wastes and apparatus therefor**

(71) Applicant: Mizuse Leather MFG. Co., Ltd., Himeji-shi, Hyogo (JP); Hiroyuki Yoneda, Himeji-shi, Hyogo (JP); Tsuyoshi Nakayasu, Himeji-shi, Hyogo (JP)
(72) Inventor: Mizuse, Tomitsugu, Mikunino-cho Himeji-shi Hyogo (JP); Mizuse, Morio, Mikunino-cho Himeji-shi Hyogo (JP); Yoneda, Hiroyuki, Himeji-shi Hyogo (JP); Nakayasu, Tsuyoshi, Himeji-shi Hyogo (JP)
(74) Representative: Böhm, Brigitte, Dipl.-Chem. Dr.

(57) **Abstract**

Tanned leather wastes are crushed in a crusher 4, put into a pressurized heating tank 1 containing an alkaline solution having a pH of 10 to 14 to be solubilized by blowing steam to a temperature of at least 70°C; the obtained slurry is fed to a neutralization and cooling tank 5 and made neutral or weakly alkaline therein, and then fed into a methane fermentation tank 2 and subjected to methane fermentation by anaerobic bacteria treatment at a temperature of about 50 to 60°C; and the gas generated is stored in a gas holder 3 and then supplied to a gas engine for driving air compressor, a boiler or a co-generation system. Chrome hydroxide that has been precipitated in the methane fermentation tank 2 is recovered via a sludge reservoir 6, while the residue liquid is treated in an aeration tank 7.

## Description

### BACKGROUND OF THE INVENTION

### 1. Field of the Invention

The present invention relates to a process for treating tanned leather wastes which generate during processes employed in tannery. More specifically, the present invention relates to methane fermentation of such wastes by microbiological treatment.

### 2. Description of the Prior Art

The process of tannery consists of preparation processes comprising water-washing, immersion in lime, unhairing and so forth of raw leather, tanning (chrome-tanning) and finishing processes comprising shaving, dyeing, stuffing and so forth. The treatment of waste water and solid wastes generated in each of these processes is very important in view of environmental safety. In particular, treatment of tanned leather wastes, i.e. trims and slice wastes, which contain chrome, can hardly be carried out by individual leather manufacturers because of high cost and hence the treatment necessarily relies on local governments. The local governments then treat the wastes by landfill, heat-drying to obtain raw materials for fertilizer, or reducing weight and making nontoxic in a gasification-fusion furnace, i.e. slug formation, or like means, all of which cost a great deal.

On the other hand, in the field of food processing and like industries, aerobic and anaerobic microbial treatments have been employed for treating wastes, of which, in particular, anaerobic treatment has been increasing because of its small energy consumption. In the field of for example marine product industries, several pretreatment processes for biological treatments have been developed, such as acceleration of degradation of wastes with an acid or alkali, oxidation with an oxidant (ozone), mechanical crushing and pulverization by application of ultrasonic wave. There has also been developed a process which comprises forming wastes into slurry and then permitting the slurry to undergo methane fermentation (biological treatment), as well as a solid fermentation process which permit the wastes to undergo fermentation in their original solid form.

As described above, treatment of tanned leather wastes (trim wastes and slice wastes), which contain chrome, must take environmental safety into consideration. It is also important to have the viewpoint of producing usable energy from the waste treatment process. Research has therefore been made to decompose the proteins and fats and oils of tannery wastes (tanned leather wastes) by biological treatment (methane fermentation) to recover methane gas. Separately, biological treatment of crushed tannery wastes as they are solid has been commercially carried out. Methane fermentation of raw leather has also been carried out.

However, the above process which, in order to carry out methane fermentation of the tanned leather wastes, comprises only crushing the wastes and then subjecting the obtained solid wastes to biological treatment has the problem that too many days (for example, 15 to 30 days) are required for decomposition (digestion). This process therefore cannot increase the efficiency of fermentation treatment for recovering methane. The above process employed for treating wastes of marine-product and like food industries, which comprises forming the wastes into slurry by simple crushing and then carrying out methane fermentation of the slurry is hardly applicable to tanned leather wastes containing various ingredients that are not contained in the food wastes. In particular, heavy metals including chrome which are contained in tannery wastes inhibit biological treatment. The above usual process comprising crushing tanned leather wastes and then subjecting the crushed wastes to biological treatment requires very long time for fermentation to recover methane.

The above process of converting tanned leather wastes into raw material for fertilizer, which causes accumulation of chrome in the soil, is not suitable for large-scale treatment. There may also be employed a process of drying and then incinerating the wastes, which should convert 3-valent chrome into toxic 6-valent chrome, cannot be said to be environmentally safe.

### OBJECTS AND SUMMARY OF THE INVENTION

In view of the above, an object of the present invention is to provide a process for enhancing the efficiency of methane fermentation of tanned leather wastes on their anaerobic microbial treatment, thereby producing energy from the wastes efficiently.

Another object of the present invention is to provide an apparatus for the above process.

In order to achieve the above first object, the present invention provides a process which comprises subjecting tanned leather wastes to methane fermentation treatment with anaerobic bacteria and recovering the methane that generates on the fermentation.

The present invention also provides, to achieve the above second object, an apparatus for treating tanned leather wastes, which comprises:
a heating tank for solubilizing tanned leather wastes by heating in an alkaline solution,
a methane fermentation tank for subjecting the solubilyzed tanned leather wastes to methane fermentation by biological treatment with anaerobic bacteria, and
a gas recovering means for recovering methane generated in the methane fermentation tank.

The process for tanned leather wastes of the present invention is characterized by subjecting the tanned leather wastes to methane fermentation with anaerobic bacteria and recovering the methane that is generated during the fermentation. The methane fermentation with anaerobic bacteria (anaerobic microbial treatment), which requires only a low level of energy consumption, realizes efficient energy production, i.e. a large amount of energy recovered with a small amount of energy required for the process. Furthermore, since methane fermentation occurs on the fats and proteins contained in the wastes, there can generate a large amount of gas having a high concentration of methane. In the process of the present invention, it is preferred, prior to methane fermentation, to solubilize the wastes in an alkali solution under heating. In this case, the heating can extinct toxic bacteria and also convert chrome into chrome hydroxide which can be recovered. The gas recovered in the process can be used as fuel or heat source (gas engine or gas turbine) for the motor for co-generation system or heat source for various purposes, and also for cooling apparatuses and for driving compressors. The gas energy can be converted into cool water, warm water or compressed air, or the methane gas itself can be converted into methanol. These conversions can minimize the storage space, so that in-house treatment becomes possible and efficient recovery and effective use of the gas become also possible.

Since proteins and fats present in the tannery wastes are less decomposable anaerobically compared to hydrocarbons, simple crushing of the wastes sometimes does not lead to achievement of satisfactory fermentation rate. It is therefore recommended to solubilize the tannery wastes before methane fermentation treatment. In the present invention, the term "to solubilize" means "to slurry", i.e. "to form into a slurry". Solubilization of the tannery wastes into slurry accelerates decomposition (digestion) with anaerobic microorganisms, thereby increasing the efficiency of methane fermentation.

The solubilization is desirably carried out by heating in an alkaline solution. It is also desirable, after the solubilization, to carry out methane fermentation in a neutral or weakly alkaline solution (digestion liquid). The heating in an alkaline solution can efficiently solubilize the tannery wastes. Neutralization after the solubilization is desirably performed to a minimum level, so that methane fermentation can be carried out in a neutral or weakly alkaline solution. This process ensures that chrome is insolubilized into nontoxic chrome hydroxide precipitates, which can then be discharged. In the digestion liquid, nontoxic 3-valent chrome is then, by reaction with a neutral or weakly alkaline solution, converted into neutral and insoluble chrome hydroxide. Since the insolubilized chrome has little effect of inhibiting methane fermentation with anaerobic bacteria, gas can generate efficiently.

The above heating is desirably carried out at a temperature of at least 70°C and in an alkaline solution having a pH of 10 to 14. Methane fermentation of the tanned leather wastes under these conditions can complete within a short period of about 3 to 7 days (10 days at the longest), while the usual process of only crushing the wastes and then subjecting the crushed wastes to biological treatment requires 15 to 30 days.

The process of treating tanned leather wastes according to the present invention may be carried out with an apparatus which comprises a heating tank for solubilizing the tanned leather wastes by heating in an alkaline solution, a methane fermentation tank for subjecting tanned leather wastes to methane fermentation by biological treatment with anaerobic bacteria, and a gas recovering means for recovering methane generated in the methane fermentation tank. The gas recovering means may be a gas holder or the like for storing the gas or a direct suppler to co-generation or like systems.

### BRIEF DESCRIPTION OF THE DRAWINGS

FIG.1 is a schematic configuration view of a system construction and operation flow of an example of the apparatus (biogas plant) for treating tanned leather wastes of aspect of an embodiment of the present invention; and
FIG.2 is a plan of the layout of the same apparatus for treating tanned leather wastes.

### DESCRIPTION OF THE PREFERRED EMBODIMENTS

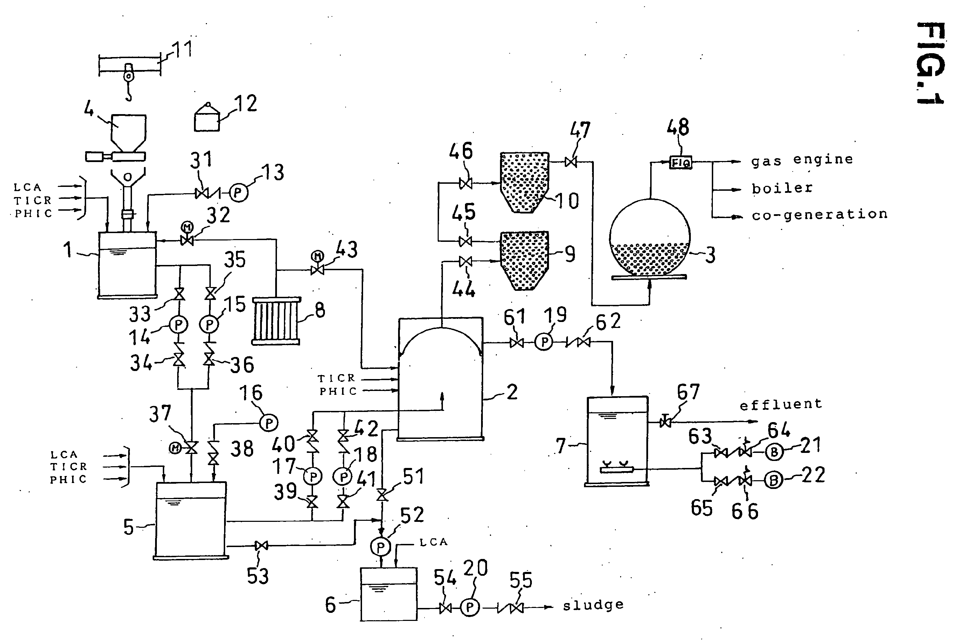

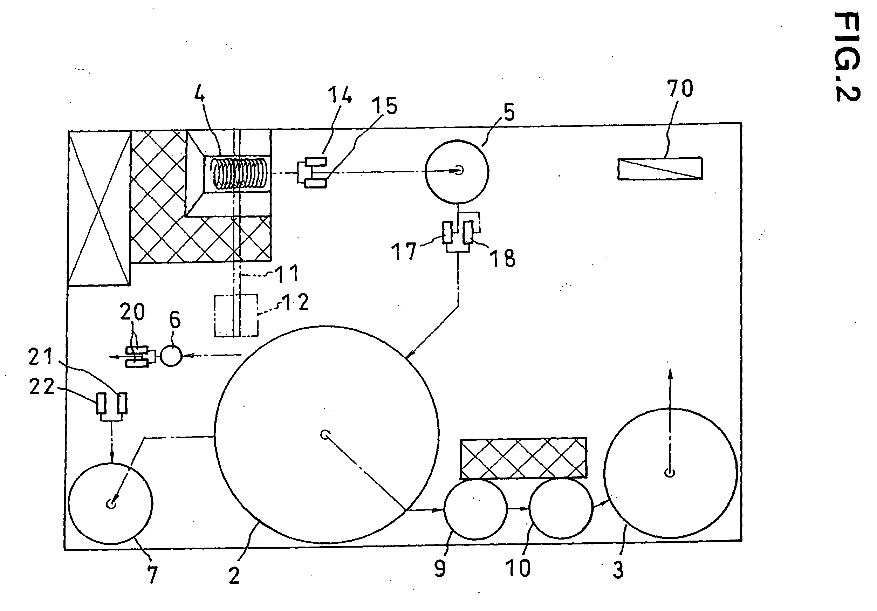

The apparatus for treating tanned leather wastes shown in FIGURES 1 and 2 is a biogas plant which treats the wastes containing chrome by methane fermentation with anaerobic bacteria, recovers the methane generated and uses the methane as fuel for a gas engine for driving a compressor and as fuel and heat source for a gas engine or gas turbine of a co-generation system. In FIGURES 1 and 2, 1 is a heating tank for solubilizing the wastes by heating in an alkaline solution; 2 is a methane fermentation tank for subjecting the solubilized wastes to biological treatment with anaerobic bacteria, thereby effecting methane fermentation; and 3 is a gas holder for recovering the methane generated. Further in the FIGURES, 4 is a crusher; 5 is a neutralization and cooling tank; 6 is a sludge reservoir; 7 is an aeration tank, 8 is a steam generator (such as a boiler); and 9 and 10 are desulfurizers. In addition, 11 is a chain block; 12 is a transporting container; 13 is an alkali injector for an alkaline solution; 14 and 15 are heating tank discharge pumps; 16 is a sulfuric acid injection pump; 17 and 18 are injection pumps for the methane fermentation tank; 19 is a residue discharge pump; 20 is a sludge transporting pump; and 21 and 22 are aeration blowers.

Tanned leather wastes such as trims and slice wastes are transported with the transporting container 12 along the chain block 11, dumped into the crusher 4 and crushed therein, and fed to the pressurized heating tank 1. Aqueous sodium hydroxide solution is injected via valve 31 through the alkali injection pump 13. The solid consistency in the heating tank 1 is then adjusted to for example about 20% and the pH of the aqueous sodium hydroxide solution in the tank to 10 to 14. Steam is then blown from the steam generator 8 via valve 32 into the tank, to heat the contents at a temperature of about 70 to 80°C for a prescribed time (e.g. about 1 hour), so that the wastes are solubilized (formed into slurry). On this solubilization treatment, the heating temperature is desirably at least 70°C , since temperatures lower than this would sometimes lead to insufficient solubilization. Higher temperatures increase the solubilization rate.

The slurry of wastes thus obtained in the heating tank 1 is withdrawn with the heating tank discharge pump 14 (pump 15 as standby) via valves 33 and 34 (valves 35 and 36 as standby) and, while the feeding rate is controlled with the electric valve 37, fed to the neutralization and cooling tank 5. Sulfuric acid is injected with the sulfuric acid solution injecting pump 16 into the neutralization and cooling tank 5 via valve 38, to neutralize slightly, thereby making the total slurry neutral or weakly alkaline.

The slurry thus made neutral or weakly alkaline is then withdrawn with the methane fermentation injection pump 17 (pump 18 as standby) via valves 39 and 40 (valves 41 and 42 as standby) and fed into the methane fermentation tank 2. Steam is blown from the steam generator 8 via the electric valve 43 to maintain the temperature desirably at about 50 to 60°C, whereby methane fermentation with anaerobic methane-producing bacteria (anaerobic bacteria) is effected.

The neutral or weakly alkaline slurry containing the solubilized tanned leather wastes is thus allowed to reside in the methane fermentation tank for a prescribed time. The gas generated is introduced via valves 44 through 47 into the desulfurizers 9 and 10. The methane gas which has been desulfurized is introduced into the gas holder 3 and stored therein. The methane is then fed, via gas meter 48, to a gas engine for driving air compressor, a boiler and a co-generation system.

On this methane fermentation, the chrome that has been contained in the tanned leather wastes is converted into chrome hydroxide and precipitated in the methane fermentation tank 2. The precipitates are withdrawn through the withdrawing pump 52 via valve 51 into the sludge reservoir 6. Residues from the neutralization and cooling tank 5 are also withdrawn via valve 53 into the sludge reservoir 6. The sludge of the precipitates and residues thus accumulated in the sludge reservoir 6 are discharged through the sludge transporting pump 20 (metering pump) via valves 54 and 55, dewatered and dumped.

Liquid remaining over the precipitates in the methane fermentation tank 2 is withdrawn through the residue discharge pump 19 via valves 61 and 62 and introduced into the aeration tank 7. Air is blown (oxygen supply) with the aeration blowers 21 and 22 via valves 63 and 64 (safety valves)(valves 65 and 66 as standby (safety valves)), thereby aerating the contents by aerobic microorganism treatment. The resulting effluent is discharged by opening valve 67.

On performing these treatments, automatic operation of each treatment is carried out on the operation panel 70 (see FIGURE 2). On this occasion, the temperature, pH and liquid level of the heating tank 1 are detected with a thermometer (TICR), a pH meter (PHIC) and a level sensor (LCA), respectively, and those of the neutralization and cooling tank 5 with a thermometer (TICR), a pH meter (PHIC) and a level sensor (LCA), respectively. The pH and liquid level of the methane fermentation tank 2 are detected with a pH meter (PHIC) and a level sensor (LCA), respectively, and the temperature of the sludge reservoir with a thermometer (TICR).

Gas recovery can thus be made from tanned leather wastes by methane fermentation with anaerobic bacteria and the recovered gas can be efficiently put into effective uses. The chrome contained in the tanned leather wastes can be made nontoxic and then precipitated and discharged, which fact permits individual leather manufacturers to treat the wastes with sufficient environmental safety.

The above embodiment uses the recovered gas as fuel for gas engines driving a compressor and for a boiler, and as fuel or heat source of a gas engine or gas turbine for a co-generation system. With respect to other uses, the recovered gas can also be used for driving cooling apparatus or like purposes, or, can be converted into cool water, hot water or compressed air, or into methanol and then stored, which minimizes the storage space.

As is clear from the above description, according to the present invention, methane fermentation of tanned leather wastes by anaerobic bacteria treatment realizes efficient recovery and effective uses of methane gas, and also can convert chrome contained in the wastes into nontoxic chrome hydroxide and precipitate the latter, thereby achieving sufficient environmental safety.

Obviously, numerous modifications and variations of the present invention are possible in light of the above teachings. It is therefore to be understood that within the scope of the appended claims, the invention may be practiced otherwise than as specifically described herein.

Tanned leather wastes are crushed in a crusher 4, put into a pressurized heating tank 1 containing an alkaline solution having a pH of 10 to 14 to be solubilized by blowing steam to a temperature of at least 70°C; the obtained slurry is fed to a neutralization and cooling tank 5 and made neutral or weakly alkaline therein, and then fed into a methane fermentation tank 2 and subjected to methane fermentation by anaerobic bacteria treatment at a temperature of about 50 to 60°C; and the gas generated is stored in a gas holder 3 and then supplied to a gas engine for driving air compressor, a boiler or a co-generation system. Chrome hydroxide that has been precipitated in the methane fermentation tank 2 is recovered via a sludge reservoir 6, while the residue liquid is treated in an aeration tank 7.

## Claims

1. A process for treating tanned leather wastes which comprises subjecting tanned leather wastes to methane fermentation treatment with anaerobic bacteria and recovering the methane that generates on the fermentation.

2. The process for treating tanned leather wastes according to Claim 1, further comprising, as a pretreatment for said methane fermentation treatment, solubilizing the tanned leather wastes.

3. The process for treating tanned leather wastes according to Claim 2, wherein the tanned leather wastes are solubilized by heat treatment in an alkaline solution and said methane fermentation is, after the solubilization, carried out in a neutral or weakly alkaline solution.

4. The process for treating tanned leather wastes according to Claim 3, wherein said heat treatment is carried out at a temperature of at least 70°C and in an alkaline solution having a pH of 10 to 14.

5. An apparatus for treating tanned leather wastes, which comprises:
a heating tank for solubilizing tanned leather wastes by heating in an alkaline solution,
a methane fermentation tank for subjecting the solubilized tanned leather wastes to methane fermentation by biological treatment with anaerobic bacteria, and
a gas recovering means for recovering methane generated in the methane fermentation tank.
